# EUROPÄISCHE PATENTSCHRIFT

(11) **EP 1 813 233 B1**
(45) Veröffentlichungstag und Bekanntmachung des Hinweises auf die Patenterteilung: **16.05.2012**
(21) Anmeldenummer: 07001590.4
(22) Anmeldetag: 25.01.2007
(51) Int. Cl.: A61F 5/01

(54) **Daumen-Orthese**
Thumb orthosis
Orthèse de pouce

(30) Priorität: 27.01.2006 DE 202006001385 U
(43) Veröffentlichungstag der Anmeldung: 01.08.2007
(73) Patentinhaber: Weskott, Christina, 50858 Köln (DE)
(72) Erfinder: Weskott, Christina, 50858 Köln (DE)
(74) Vertreter: Schäfer, Horst

(56) Entgegenhaltungen:
- DE-U1- 8 806 792
- US-A- 4 270 528
- US-A- 4 297 992
- US-A- 4 732 142
- US-A- 5 232 436

## Beschreibung

Die Erfindung betrifft eine Daumen-Orthese zur Entlastung des Daumensattelgelenks und zur Stabilisierung des Daumengrundgelenks.

Eine Daumen-Orthese ist im Stand der Technik bekannt. Sie kann bei Knochen- beziehungsweise Gelenkerkrankungen des Daumens zur Stabilisierung und Stützung bestimmter Gelenke eingesetzt werden. Daumen-Orthesen werden insbesondere bei Rhiz-Arthrose, Instabilitäten des Daumengrundgelenks oder nach Operationen zur Wiederherstellung der korrekten Gelenkstellung oder zur Minderung funktioneller Beschwerden bei Gelenkfehlstellung im Daumensattelgelenk eingesetzt.

Bei Rhiz-Arthrose handelt es sich um degenerative Prozesse am Daumensattelgelenk, wobei die Ursache in einer Schwächung der Bänder vermutet wird. Rhiz-Arthrose äußert sich durch Subluxation (nicht vollständige Verstauchung) des Metakarpale I proximal aufgrund der Instabilität, kompensatorische Hyperextension (ausgleichende Überstreckung), eingeschränkter Abduktion (heranziehende Gliedmaßenbewegung), übermäßige Flexion (Abknicken) des Daumengrundgelenks beim Greifvorgang sowie erschwerte Extension und Opposition.

Die Daumen-Orthese des Standes der Technik weist einen Ring auf mit einer Ober- und einer Unterseite. Der Daumen wird durch den Ring gesteckt. Dabei wird der Ring so über den Daumen geschoben, dass die Oberseite auf der der Handaußenfläche zugeordneten Außenfläche des Daumens anliegt. Die Unterseite liegt dann auf der der Handinnenfläche zugeordneten Innenfläche des Daumens an. Auf der Ober- und Unterseite sind Ösen vorgesehen, in welchen ein Befestigungsband schlaufenartig eingefädelt ist, wobei das Handgelenk in die Schlaufe einführbar ist und der Ring durch Festziehen des Befestigungsbandes am Handgelenk gespannt und befestigt wird.

Die Daumen-Orthese des Standes der Technik hat den Nachteil, dass der Daumen in seiner Beweglichkeit stark eingeschränkt wird. Er wird zum einen in eine ständig gespreizte Haltung gezwungen. In dieser Haltung ist die Funktion des Daumens beim Greifen ausschließlich auf einen sogenannten Spitzgriff eingeschränkt, bei welchem nur noch die Daumenkuppe an der Greifffunktion der Hand beteiligt ist, was zu einer ungewohnten Belastung beteiligter Gelenke und damit verbundenen Schmerzen führen kann. Zum anderen hat sich erwiesen, dass die Daumen-Orthese des Standes der Technik lediglich dazu geeignet ist, den Daumen abzuspreizen. Eine Stützung oder Stabilisierung der erkrankten Gelenke wird damit nicht bewirkt.

Außerdem ist gefunden worden, dass bei einem Griff mit angelegter Daumen-Orthese nach dem Stand der Technik, bei welchem der Daumen über einen bestimmten Winkel hinaus abgespreizt wird, das Daumengrundgelenk ohne die erzwungene Abspreizung ist. Das Daumensattelgelenk gerät dabei ungeschützt in seine krankhafte Fehlstellung zurück und wird in der Fehlstellung belastet, obwohl die Daumen-Orthese getragen wird.

Des Weiteren wird das Befestigungsband schnell schmutzig, da es im Bereich des Hand- oder Daumenballens unmittelbar auf der Grifffläche der Hand aufliegt und somit bei fast allen manuellen Tätigkeiten mit verschmutzenden Stoffen in Kontakt geraten kann. Eine Daumen-Orthese gemäß dem Oberbegriff des Anspruchs 1 ist aus DE 88 06 792 U1 bekannt. Hierdurch wird das Befestigungsband schnell unhygienisch und unansehnlich.

Der Erfindung liegt die Aufgabe zugrunde, die Nachteile des Standes der Technik zu überwinden, insbesondere eine Daumen-Orthese zur Verfügung zu stellen, mit welcher eine dauerhafte Stabilisierung des Daumens und eine Entlastung kranker Gelenke gewährleistet und auf Bandbefestigungen verzichtet werden kann.

Eine Lösung der erfindungsgemäßen Aufgabe wird bereitgestellt durch eine Daumen-Orthese gemäß den Merkmalen des Anspruchs 1.

Mit der erfindungsgemäßen Daumen-Orthese ist somit einer Gelenkeinfassung des Daumengrundgelenks auf der Außenfläche des Daumens möglich, wobei das Daumengrundgelenk durch den Steg, welcher auf der Innenfläche des Daumens anliegt, in einer leicht gebeugten Haltung gehalten wird. Dadurch wird das Daumengrundgelenk in die physiologisch richtige Position gedrückt und die Position des Daumensattelgelenks ausschließlich bei Belastung durch Greifen dahingehend korrigiert, dass es seinerseits in die physiologisch richtige Position gedrückt wird. Das Daumensattelgelenk, liegt dabei außerhalb der Daumen-Orthese und wird ausschließlich gemäß der Kräfteverteilung zwischen der Daumen-Orthese bzw. dem damit gestützten Daumengrundgelenk und dem Handgelenk beaufschlagt. Damit wird das Daumengrundgelenk mit der Orthese in einer leichten Beuge gehalten, womit verhindert wird, dass es in eine Überstreckung gerät, welche mit Schmerzen verbunden sein kann.

Der Steg der Orthese, welcher am Daumengrundgelenk an der Innenfläche des Daumen angeordnet ist, drückt dieses nach außen in die physiologische Position. Dadurch wird erreicht, dass das Daumensattelgelenk seinerseits positiv korrigiert wird, womit eine Entlastung des Daumensattelgelenks und eine Reduzierung beziehungsweise Vermeidung von Schmerzen einhergeht.

Mit der erfindungsgemäßen Daumen-Orthese wird somit eine Korrektur von Fehlstellungen des Daumengrundgelenks und Daumensattelgelenks erreicht, ohne die Hand beziehungsweise den Daumen im unbelasteten Zustand permanent zu beaufschlagen beziehungsweise in einer gespreizten Haltung zu halten. Dabei wird ein Einknicken des Daumengrundgelenks und die damit verbundene schmerzhafte Überstreckung des Daumensattelgelenks verhindert.

Darüber hinaus kann der Daumen um den Steg herum ohne wesentlich beziehungsweise bemerkbare Einschränkungen gebeugt werden und bleibt in vorteilhafter Weise vollständig und weitgehend schmerzfrei einsetzbar. Der Daumen kann vollständig beim Greifen eingesetzt werden, wobei die Orthese ohne eine Behinderung dazustellen, am Daumen verbleiben kann und am Daumen eigenständig gehalten wird, d.h. die Orthese rutscht auch ohne Bandbefestigung nicht vom Daumen ab.

Da die Daumen-Orthese unablässig getragen werden kann, kann einem Fortschreiten der degenerativen Umbauprozesse im Bewegungsapparat des Daumens entgegen gewirkt werden. Dies ermöglicht, dass sowohl die Symptome wie beispielsweise Schmerzen gemindert werden können als auch ein Rückbauprozess an den Gelenken und Knochen ermöglicht wird, mit welchem Fehlbildungen rückgebildet werden können.

Eine Ausführung der erfindungsgemäßen Daumen-Orthese wird dadurch bereitgestellt, dass der untere Ringbügel eine zum Daumensattelgelenk auslaufende Stützfläche aufweist. Die Stützfläche ist in vorteilhafter Weise vom Steg ausgehend verbreitert, so dass der Daumenstumpf und der Rand des Hand- bzw. Daumenballens von der Orthese umschlossen ist. Dabei ist vorgesehen, dass die Stützfläche zumindest zur Daumeninnenfläche offen ist, so dass der Daumen in Greifrichtung um den Steg beugbar und somit vollständig bewegbar bleibt. Bei höherem Grad der Gelenkerkrankung kann die Dimensionierung der Stützfläche größer ausfallen, während bei leichten Erkrankungen eine kleine Stützfläche ausreicht.

Des Weiteren wird eine Ausführungsform dadurch bereitgestellt, dass der Steg zumindest teilweise zwischen dem oberen Ringbügel und dem unteren Ringbügel als verbreiterte integrierte Stützschiene ausgebildet ist. Die Stützschiene verläuft dabei vom oberen Ringbügel aus, wobei der obere Ringbügel am Daumen distal anliegt. Distal bedeutet in diesem Zusammenhang herzfern an dem Daumen, also zum Daumennagel hin. Außerdem umschließt der Ringbügel den Daumen auf der Außenfläche des Daumens.

Von der Erfindung umfasst ist außerdem eine Ausführungsform, bei der welcher die Stützschiene bis zum Ende des unteren Ringbügels verläuft. Erfindungsgemäß ist dabei vorgesehen, dass die Stützschiene den handinneren Rand des Daumens umschließt, d.h. die Stützschiene liegt an der Seite der Hand an, welche zum Körper gerichtet ist.

In vorteilhafter Weise wird dadurch erreicht, dass das Daumengrundgelenk am Hand- bzw. Daumenballen geschient ist. Die Stützwirkung der Daumen-Orthese wird somit verbessert, da sich die Stützfläche weiter über die angrenzende Stützschiene erstreckt. Zudem erhält der Daumen eine weitere Stützung am innenliegenden Daumenrand, was zu einer weiteren Stabilisierung des Bewegungsapparates des Daumens führt. Bei höherem Grad der Gelenkerkrankung kann die Dimensionierung der Stützschiene größer ausfallen, während bei leichten Erkrankungen eine kurze Schienung ausreicht.

Dabei ist vorgesehen, dass die Stützfläche und die Stützschiene integral ineinander geformt sind, so dass eine ergonomisch günstige Anlage an den Daumen möglich ist.

Erfindungsgemäß ist vorgesehen, dass die Daumen-Orthese durch Gelenkmittel an einem Ringbügel im Bereich des Stegs aufklappbar und mittels Schließen im geschlossenen Zustand arretierbar ist. Eine derartige Ausgestaltung der Orthese kann bei dem Krankheitsbild eines sogenannten 90/90-Daumen eingesetzt werden, welches auch als Schuster-Daumen bekannt ist. Dabei ist eine Form gefunden worden, bei welcher die Daumen-Orthese im geschlossenen Zustand eine ergonomisch optimale Anlagefläche aufweist. Die Daumen-Orthese weist dazu Scharniermittel und wenigstens eine Schließe auf. auf. Im geschlossenen Zustand werden die Ringbügel mittels Schließen zusammengehalten. Werden die Schließen geöffnet, können die Ringbügel aufgeklappt werden, so dass dem Daumen ein größerer Öffnungsquerschnitt beim Einführen zur Verfügung steht, wie dies zum Beispiel bei einem 90/90-Daumen (Schuster-Daumen) erforderlich ist. In vorteilhafter Weise ist vorgesehen, dass zum einen Steg und Ringbügel bewegbar sind. Bei dieser Ausführungsform sind die beiden Ringbügel zueinander fest und die Scharniermittel sind zwischen dem Steg und der verbleibenden Orthese angeordnet.

Alternativ können die Scharniermittel erfindungsgemäß an einem Ringbügel angeordnet sein. Bei dieser bevorzugten Ausführungsform ist vorgesehen, dass der Steg einstückig mit zumindest einem Ringbügel verbunden ist, vorzugsweise mit dem oberen Ringbügel. Der andere Ringbügel ist demgegenüber entsprechend klappbar gehalten. Dabei bildet der Steg zusammen mit dem Ringbügel einen Grundkörper und der weitere Ringbügel ist auf jeder Seite mit Scharnieren an diesem Grundkörper befestigt. Schließen sitzen seitlich an der Daumen-Orthese. Dabei sind konventionelle Schließen einsetzbar, welche aus der Schmuckherstellung bekannt sind.

In einem Ausführungsbeispiel der Erfindung ist weiter vorgesehen, dass die Ringbügel zur Gewährleistung der einseitigen Daumenbeweglichkeit daumeninnenseitig offen ausgebildet sind. Die Daumen-Orthese ist dabei so ausgebildet, dass der Steg die Innenfläche des Daumens ergonomisch günstig geformt umgreift. Die Ringbügel erstrecken sich vom Steg ausgehend außen um den Daumen, wobei sie sich nach außen hin flächig verbreitern und auf beiden Seiten zum Steg hin verengen. Dabei liegt der obere Ringbügel distal und der untere Ringbügel proximal am Daumengrundgelenk. Dabei stehen die durch die Ringbügel und den Steg für die Daumenöffnungen gebildeten Ebenen, durch welche der Daumen in die Orthese eingeführt wird, im Wesentlichen v-förmig aufeinander und schneiden sich im Bereich des Stegs.

In vorteilhafter Weise ist vorgesehen, dass die Daumen-Orthese oder zumindest einzelne Bauelemente der Daumen-Orthese aus Kunststoff, Metall, keramischem Material oder Verbundwerkstoff bestehen. Somit sind Daumen-Orthesen herstellbar, welche chemisch resistent und somit leicht zu reinigen sind. Darüber hinaus ist es möglich die Daumen-Orthese mit Materialien herzustellen, welche hohe Temperatur- und Formstabilität aufweisen. Es ist von der Erfindung ebenfalls eine Daumen-Orthese aus Material umfasst, welches schmutzabweisende Eigenschaften aufweist.

Des Weiteren ist vorgesehen, dass zumindest flächenhafte Bereiche einzelner Bauelemente der Daumen-Orthese filigrane Ausnehmungen oder ergonomische Ausformungen aufweisen. Hierdurch ist es in vorteilhafter Weise möglich, das Gewicht der Daumen-Orthese wesentlich zu reduzieren, was den Tragekomfort insbesondere beim Dauertragen, verbessert. Außerdem wird eine vessere Belüftung unter der daumen-Orthese erreicht.

Die Erfindung wird im Folgenden anhand der Zeichnungen von Ausführungsbeispielen der Daumen-Orthese erläutert. Es zeigen
- Figur1: eine nicht erfindungsgemäße Daumen-Orthese in der vorgesehenen Trageposition am Daumen;
- Figur 2: eine perspektivische Draufsicht auf die nicht erfindungsgemäße Daumen-Orthese;
- Figur 3: die Seitenansicht einer erfindungsgemäßen Daumen-Orthese mit zwei aufklappbaren Teilstücken und Gelenkmitteln im geschlossenen Zustand.

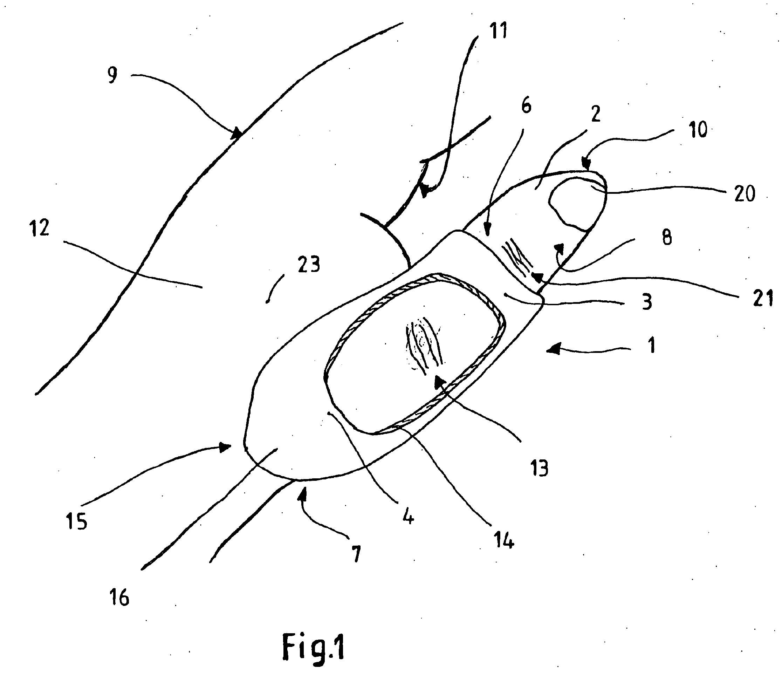

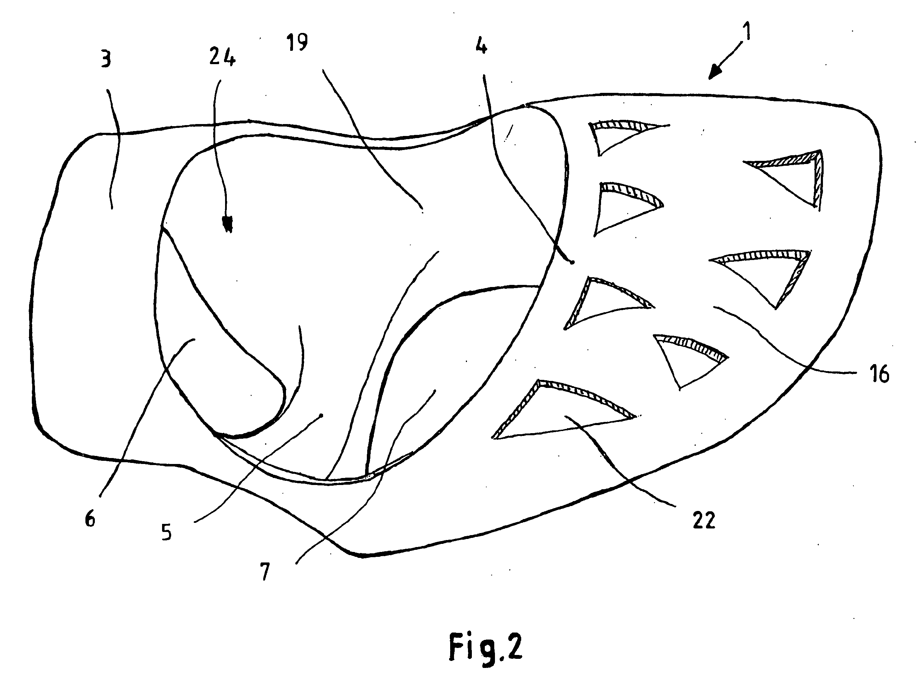

Figur 1 zeigt eine nicht erfindungsgemäße Daumen-Orthese 1 in der vorgesehenen Trageposition am Daumen 2. Die Daumen-Orthese 1 weist einen oberen Ringbügel 3, einen unteren Ringbügel 4 sowie einem Steg 5 auf. Wie in Figur 2 sichtbar bildet der Steg 5 mit jedem der beiden Ringbügel 3, 4 eine ringförmige Öffnung 6, 7, durch welche der Daumen 2 beim Anlegen der Daumen-Orthese 1 nacheinander durchgesteckt werden kann. Die Ringbügel 3, 4 liegen in dem in Figur 1 dargestellten, angelegten Zustand der Orthese auf der Außenfläche 8 des Daumens 2, welche der Handaußenfläche 9 zugeordnet ist, während der Steg 5 auf der entgegengesetzten Innenfläche 10 des Daumens 2 anliegt, welche der Handinnenfläche 11 zugeordnet ist. Der Steg 5 ist in Figur 1 vom Daumen 2 verdeckt und daher nicht sichtbar.

Die Daumen-Orthese 1 sitzt durch das Zusammenwirken der beiden Ringbügel 3, 4 auf der Außenfläche 8 mit dem Steg 5 auf der Innenfläche 10 des Daumens 2 in der Trageposition. Die Trageposition wird dabei durch die Endlage des Stegs 5 in der Beuge des Daumens 2 zwischen Hand 12 und Daumen 2 am Daumengrundgelenk 13 festgelegt.

Die Daumen-Orthese 1 ist in der Trageposition somit am Daumengrundgelenk 13 fest angeordnet, wobei die beiden Ringbügel 3, 4 eine Gelenkeinfassung 14 bilden, welche das Daumengrundgelenk 13 auf der Außenfläche 8 des Daumens 2 umfasst. Das Daumengrundgelenk 13 bleibt somit bei einer nach Innen zur Innenfläche 10 des Daumens 2 gerichteten Beugung frei und vollständig beweglich. Nach Außen hin wird das Einknicken der das Daumengrundgelenk 13 bildenden Gliedmaßen durch die starre Anordnung der Ringbügel 3, 4 im Verhältnis zum Steg 5 verhindert, zwischen welchen der Daumen 2 einliegt.

In vorteilhafter Weise wird mit der Daumen-Orthese 1 somit eine Stützfunktion des Daumengrundgelenks 13 gewährleistet, ohne dass die Daumen-Orthese 1 vom Daumengrundgelenk 13 und somit auch vom Daumen 2 abrutschen kann, wobei auf Befestigungsbänder verzichtet werden kann.

Ein besonderer Vorteil der Erfindung stellt sich dadurch dar, dass die gesamte Hand 12 greiffähig bleibt. Dabei werden die das Daumengrundgelenk 13 bildenden Gliedmaßen durch die starre Anordnung der Ringbügel 3, 4 in einer leichten Beuge gehalten, so dass beim Greifen der Hand 12 der am Daumensattelgelenk 15 wirksame Kraftwinkel stets ausreichend klein bleibt und somit die das Daumensattelgelenk 15 bildenden Gliedmaßen ebenfalls in einer leichten Beuge bleiben. Damit wird das Daumensattelgelenk 15 stabilisiert.

Die Stützfunktion der Daumen-Orthese 1 am Daumensattelgelenk 15 wird durch eine am unteren Ringbügel 4 ausgebildete Stützfläche 16 unterstützt. Die Stützfläche 16 erstreckt sich als einstückig angeformte Verbreiterung des unteren Ringbügels 4 auf der Außenfläche 8 des Daumen 2 in Richtung des Daumensattelgelenks 15 und ist mit einer konkaven Wölbung an die Ergonomie der Hand 12 angepasst.

Die Bewegungsfreiheit des Daumen 2 wird dabei weiterhin durch die Gelenkeinfassung 14 gewährleistet, welche ein Ausweichen des Daumengrundgelenks 13 beim Beugen ermöglicht.

In Figur 2 ist eine Daumen-Orthese 1 in perspektivischer Draufsicht von oben gezeigt, wodurch die Anordnung des Steges 5 im Verhältnis zu den Ringbügeln 3, 4 verdeutlicht wird. Der obere Ringbügel 3 bildet mit dem Steg 5 eine obere Öffnung 6 und mit dem unteren Ringbügel 4 eine untere Öffnung 7. Der untere Ringbügel 4 ist zu der Stützfläche 16 verbreitert ausgebildet.

An der Daumen-Orthese 1 ist der Steg 5 zwischen dem oberen Ringbügel 3 und dem unteren Ringbügel 4 als integrierte Stützschiene 19 ausgebildet. Dazu sind die Ringbügel 3, 4 auf der zum Körper liegenden Seite des Daumen 2 um das Daumengrundgelenk 13 verbreitert ausgebildet. Das Daumengrundgelenk 13 wird somit zusätzlich beim Greifen der Hand in der Bewegungs- beziehungsweise Beugungsebene gehalten. Die Bewegungsbeziehungsweise Beugungsebene wird durch die Gelenkachse des Daumengrundgelenks 13 festgelegt und ist in der Figur 2 nicht dargestellt.

Beim Anziehen der Daumen-Orthese 1 wird der Daumen 2 zunächst in die untere Öffnung 7 zwischen Steg 5 und unterem Ringbügel 4 eingeschoben und durch leichtes Beugen weitergeschoben. Dabei passiert die Daumenspitze 20 sowie das Daumenendgelenk 21 die Gelenkeinfassung 14 und wird im Folgenden von Innen durch die obere Öffnung 6 zwischen Steg 5 und oberen Ringbügel 3 aus der Daumen-Orthese 1 herausgeschoben. Das Daumengrundgelenk 13 wird dabei in die Gelenkeinfassung 14 eingeführt, so dass der Steg 5 auf der Innenfläche 10 des Daumengrundgelenks 13 in eine stabile Position gelangt. Dabei liegt der obere Ringbügel 3 unterhalb oder auf dem Daumenendgelenk 21. Oben wird im Zusammenhang mit der erfindungsgemäßen Daumen-Orthese 1 stets im Sinne von körperfern (distal) und unten im Sinne körpernahe (proximal) verwendet.

Die dargestellte nicht erfindungsgemäße Daumen-Orthese 1 ist einstückig ausgebildet und die vorbeschriebenen Bestandteile der Ringbügel 3, 4, der Stützschiene 19 und des Steges 5 integriert. Damit der Tragekomfort gewährleistet ist, ist vorgesehen, dass zumindest flächenhafte Bereiche einzelner Bauelemente der Daumen-Orthese 1 filigrane Ausnehmungen 22 aufweisen. Diese können auch als ergonomische Ausformungen auf den Flächen ausgebildet sein.

Die dargestellte Daumen-Orthese 1 hat Ausnehmungen 22 auf der integrierten Stützfläche 16, welche verteilt sind bis zur Stützschiene 19. Alternativ oder ergänzend ist es möglich, dass die Ausnehmungen 22 auch auf der Stützschiene 19 angeordnet sind. Dabei kann die Anordnung beliebig gewählt sein. Insbesondere lassen sich durch die Anordnung der Ausnehmungen 22 in vorteilhafter Weise Designwirkung nach dem aktuellen ästhetischen Formenschatz realisieren. Die Ausnehmungen 22 haben dabei den Vorteil, dass die abgedeckten Bereiche der Haut 23 beim Tragen, insbesondere in den Bereichen der Stützfläche 16 und der Stützschiene 19 belüftet bleiben. Somit kann die Daumen-Orthese 1 auch bei üblichen Handhabungen im Haushalt und Beruf getragen werden, wobei das Arbeiten mit Schmutz und Flüssigkeiten ohne Probleme möglich bleibt. Die Hand 12 kann ohne Ablegen der Daumen-Orthese 1 gewaschen werden, wobei Schmutzansammlungen zwischen Haut 23 und der Innenfläche 24 der Daumen-Orthese 1 durch die Ausnehmungen 22 weitgehend unterbleiben. Vielmehr fließt Wasser durch die Ausnehmungen 22 zwischen die Daumen-Orthese und die Haut 23 und trägt Schmutz und Verunreinigungen gegebenenfalls weg.

Des Weiteren trocknet dieser Bereich, der von einem Handtuch nur unzureichend erreicht werden kann, durch die mit den Ausnehmungen 22 erreichte Belüftung schnell. In vorteilhafter Weise bleibt der Bereich zwischen Haut 23 und der Innenfläche 24 der Daumen-Orthese 1 somit hygienisch.

Selbst das Einfetten der Hände 12 bleibt bei angelegter Daumen-Orthese 1 gemäß der Erfindung problemlos möglich, da Handkosmetika durch die Gelenkeinfassung 14 sowie die Ausnehmungen 22 zur Haut 23 gelangen können und sich unter der Daumen-Orthese bei üblicher Handmotorik leicht verteilen.

Die Daumen-Orthese 1 ist zudem in vorteilhafter Weise nach Benutzung aufgrund der offenen Ausgestaltung einfach und hygienisch einwandfrei zu reinigen.

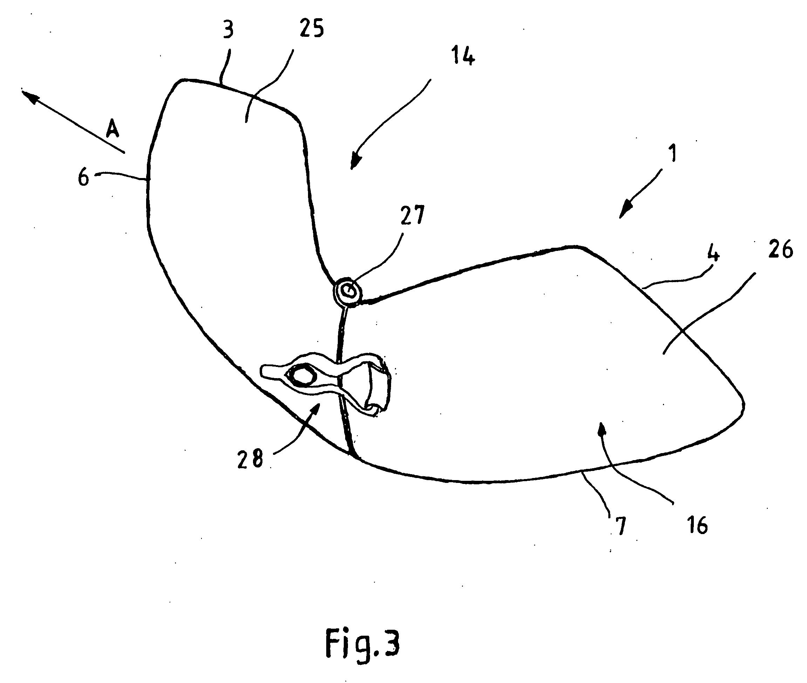

Figur 3 zeigt die Seitenansicht einer erfindungsgemäßen Daumen-Orthese 1 mit zwei aufklappbaren Teilstücken 25, 26. Die dargestellte Daumen-Orthese 1 weist dabei eine Gelenkachse senkrecht zur Einschubrichtung (Pfeil A) des Daumen 2 auf.

Die Teilstücke 25, 26 weisen Gelenkmitteln 27 auf und sind im geschlossenen Zustand dargestellt. Die Gelenkmittel 27 umfassen auf jeder Seite der Gelenkeinfassung 14 eine an Schmuckstücken übliche Scharnierverbindung 27. Ebenfalls auf jeder Seite der Gelenkeinfassung 14 ist eine entsprechende Schließe 28 angeordnet, um die Daumen-Orthese 1 im geschlossenen Zustand zu halten.

Die Daumen-Orthese 1 ist nach dem Öffnen der Schließen 28 aufklappbar, wobei die obere und untere Öffnung 6, 7 weitgehend parallel übereinander angeordnet werden können. Somit kann die Daumen-Orthese 1 in einfacher Weise auch bei einem krankhaft gekrümmten Daumen 2 (90/90-Daumen, Schuster-Daumen) angelegt und getragen werden, ohne dass schmerzhafte Zwangsbewegungen hierfür erforderlich sind. Dabei bleibt es ohne Belang für die von der Erfindung erzielten Vorteile, ob der Steg 5 nun mit dem oberen Teilstück 25 oder mit dem unteren Teilstück 26 einstückig verbunden ist.

Alternative (nicht dargestellte) Ausführungsformen der aufklappbaren Daumen-Orthese 1 können dadurch bereitgestellt werden, dass die Daumen-Orthese 1 längs der Einschubrichtung des Daumen 2 in zwei Teilstücke 25, 26 geteilt und aufklappbar ist. Dabei kann die Schließe 28 unmittelbar am Steg 5 angeordnet sein und die Gelenkmittel 27 oben auf den Ringbügeln 3, 4 oder umgekehrt, zumindest ein Gelenkmittel 27 am Steg 5 und die Schließen 28 oben auf den jeweiligen Ringbügeln 3, 4.

Die Teilstücke 25, 26 der Daumen-Orthese 1 werden dabei wie zwei Flügel aufgeklappt und der Daumen 1 eingelegt. Anschließend können die Teilstücke 25, 26 wieder zusammengeklappt und mittels der Schließen 28 arretiert werden.

Eine weitere von der Erfindung umfasste Alternative wird dadurch bereitgestellt, dass der Steg 5 aufklappbar ausgestaltet ist, beispielsweise der Steg 5 mit Gelenkmittel 27 und Schließen 28 versehen ist.

Von Vorteil ist dabei, dass auch Daumen 2 mit krankhaften Versteifungen in der erfindungsgemäßen Daumen-Orthese 1 aufgenommen werden können. Somit ist es möglich den Daumen einerseits zu stützen und andererseits den Bewegungsapparat zu verbessern. Die Bewegungsvielfalt des Daumen 2 in der Daumen-Orthese ermöglicht es dabei, den Daumen 2 entweder gestützt zu therapieren oder den Daumen 2 trotz einer gegebenen eingeschränkten Daumenmotorik bei den üblichen Handgriffen gestützt mitzubewegen, wodurch eine Verbesserung der Beweglichkeit und eine Minderung der Versteifung erreichbar ist.

Von der Erfindung ist ebenfalls umfasst, dass die Daumen-Orthese oder zumindest einzelne Bauelemente der Daumen-Orthese aus Kunststoff, Metall, keramischem Material oder Verbundwerkstoff bestehen. Dabei können für die Daumen-Orthese 1 Werkstoffe nach funktionellen Eigenschaften, wie dauerhafter optischer Haltbarkeit, chemischer oder physikalischer (Licht, Temperatur) Beständigkeit, Gewicht sowie Reinigungsfähigkeit und Handhabbarkeit eingesetzt werden. Aber es ist auch möglich hierfür Werkstoffe nach ästhetischen Gesichtspunkten auszuwählen. Kombinationen von Werkstoffen können dabei vorteilhafte Synergien der Wirkungen erzeugen, welche ebenfalls von der Erfindung umfasst sind. Bevorzugt werden Edelmetalllegierungen wie beispielsweise Gold- beziehungsweise Silberlegierungen verwendet.

Mit der erfindungsgemäßen Daumen-Orthese 1 wird erreicht, dass der Daumen 2 während des Tragens in einer physiologischen Stellung verbleiben kann. Es wird insbesondere vermieden, dass der Daumen 2 unnatürlich und zwangsweise abgespreizt gehalten wird, was mit erheblichen Einschränkungen der Bewegungsfähigkeit verbunden wäre. Somit bleiben die Bewegungsmöglichkeiten des Daumen 2 erhalten, so dass die Muskulatur des Daumen 2 weiter gefordert wird und ein kontraproduktiver Muskelabbau vermieden werden kann.

Beim Tragen der erfindungsgemäßen Daumen-Orthese 1 ist eine dauerhafte Stabilisierung des Daumens 2 und eine Entlastung kranker Gelenke gewährleistet. Dabei kann erfindungsgemäß auf störende Bandbefestigungen verzichtet werden.

### Bezugszeichenliste

- 1: Daumen-Orthese
- 2: Daumen
- 3: oberer Ringbügel
- 4: unterer Ringbügel
- 5: Steg
- 6: obere ringförmige Öffnung
- 7: untere ringförmige Öffnung
- 8: Außenfläche des Daumens
- 9: Handaußenfläche
- 10: Innenfläche des Daumens
- 11: Handinnenfläche
- 12: Hand
- 13: Daumengrundgelenk
- 14: Gelenkeinfassung
- 15: Daumensattelgelenk
- 16: Stützfläche
- 19: integrierte Stützschiene
- 20: Daumenspitze
- 21: Daumenendgelenk
- 22: Ausnehmungen
- 23: Haut
- 24: Innenfläche der Orthese
- 25: oberes Teilstück
- 26: unteres Teilstück
- 27: Scharnierverbindung/ Gelenkmittel
- 28: Schließe
- Pfeil A: Einschubrichtung des Daumen

## Patentansprüche

1. Daumen-Orthese (1) mit einem oberen distalen und einem unteren proximalen Ringbügel (3,4) und einem Steg (5), wobei der Steg (5) mit jedem der beiden Ringbügel (3,4) eine ringförmige Öffnung (6,7) zur Einführung des Daumens (2) bildet, wobei beide Ringbügel (3,4) eine Gelenkeinfassung (14) bilden, welche das Daumengrundgelenk (13) auf der Handaußenfläche (9) umschließt und dass die Daumen-Orthese (1) durch das Zusammenwirken beider Ringbügel (3,4) auf der der Handaußenfläche (9) zugeordneten Außenfläche (8) des Daumens (2) mit dem Steg (5) auf der der Handinnenfläche (11) zugeordneten Innenfläche (10) des Daumens (2) in einer Endlage am Daumengrundgelenk (13) festlegbar ist,
**dadurch gekennzeichnet, dass**
die Daumen-Orthese (1) durch Gelenkmittel (27) an einem Ringbügel (3,4) im Bereich des Stegs (5) aufklappbar und mittels Schließen (28) im geschlossenen Zustand arretierbar ist.

2. Daumen-Orthese (1) gemäß Anspruch 1,
**dadurch gekennzeichnet,**
**dass** der untere Ringbügel (4) eine zum Daumensattelgelenk (15) auslaufende Stützfläche (16) aufweist.

3. Daumen-Orthese (1) gemäß einem der Ansprüche 1 oder 2,
**dadurch gekennzeichnet,**
**dass** der Steg (5) zumindest teilweise zwischen dem oberen Ringbügel (3) und dem unteren Ringbügel (4) als verbreiterte integrierte Stützschiene (19) ausgebildet ist.

4. Daumen-Orthese (1) gemäß Anspruch 1,
**dadurch gekennzeichnet,**
**dass** der Steg (5) einstückig mit zumindest einem Ringbügel (3,4) verbunden ist, vorzugsweise mit dem oberen Ringbügel (3).

5. Daumen-Orthese (1) gemäß einem der Ansprüche 1 bis 4,
**dadurch gekennzeichnet,**
**dass** die Ringbügel (3,4) zur Gewährleistung der einseitigen Daumenbeweglichkeit daumeninnenseitig offen ausgebildet sind.

6. Daumen-Orthese (1) gemäβ einem der Ansprüche 1 bis 5,
**dadurch gekennzeichnet,**
**dass** die Daumen-Orthese (1) oder zumindest einzelne Bauelemente der Daumen-Orthese (1) aus Kunststoff, Metall, keramischem Material oder Verbundwerkstoff bestehen.

7. Daumen-Orthese (1) gemäß einem der Ansprüche 1 bis 6,
**dadurch gekennzeichnet,**
**dass** zumindest flächenhafte Bereiche einzelner Bauelemente der Daumen-Orthese (1) filigrane Ausnehmungen (22) oder ergonomische Ausformungen aufweisen.

## Claims

1. Thumb orthosis (1) comprising an upper distal and a lower proximal annular bow (3, 4) and a web (5), wherein the web (5) forms with each of the two annular bows (3, 4) an annular opening (6, 7) for the insertion of the thumb (2), wherein the two annular bows (3, 4) form an articulation frame (14) which encloses the thumb base joint (13) on the outer surface (9) of the hand, and wherein the thumb orthosis (1) is locatable in an end position at the thumb base joint (13) by the interaction of the two annular bows (3, 4) on the outer surface (8) of the thumb (2) which is assigned to the outer surface (9) of the hand with the web (5) on the inner surface (10) of the thumb (2) which is assigned to the inner surface (11) of the hand,
**characterised in that**
the thumb orthosis (1) can be folded upwards by articulation means (27) at an annular bow (3, 4) in the region of the web (5) and locked in the closed state by means of closures (28).

2. Thumb orthosis (1) according to claim 1,
**characterised in that**
the lower annular bow (4) has a support surface (16) which runs out towards the thumb saddle joint (15).

3. Thumb orthosis (1) according to claim 1 or 2,
**characterised in that**
the web (5) is at least partially configured as a widened support rail (19) between the upper annular bow (3) and the lower annular bow (4).

4. Thumb orthosis (1) according to claim 1,
**characterised in that**
the web (5) is integrally joined to at least one annular bow (3, 4), preferably to the upper annular bow (3).

5. Thumb orthosis (1) according to any of claims 1 to 4,
**characterised in that**
the annular bows (3, 4) are open on the inside of the thumb to ensure one-sided thumb mobility.

6. Thumb orthosis (1) according to any of claims 1 to 5,
**characterised in that**
the thumb orthosis (1) or at least individual components of the thumb orthosis (1) is/are made of plastic, metal, a ceramic material or a composite.

7. Thumb orthosis (1) according to any of claims 1 to 6,
**characterised in that**
at least planar regions of individual components of the thumb orthosis (1) have filigree cut-outs (22) or ergonomic shapings.

## Revendications

1. Orthèse (1) de pouce dotée d'étriers annulaires (3, 4) supérieur distal et inférieur proximal et d'une partie médiane (5), la partie médiane (5) formant avec chacun des deux étriers annulaires (3, 4) une ouverture circulaire (6, 7) pour l'introduction du pouce (2), les deux étriers annulaires (3, 4) formant un bord articulé (14) lequel entoure l'articulation métacarpophalangienne (13) du pouce sur le côté du dos (9) de la main, l'orthèse (1) de pouce peut être engagée dans une position finale sur l'articulation métacarpophalangienne (13) du pouce par l'interaction des deux étriers annulaire (3, 4) sur le côté extérieur (8) du pouce (2) adjacent au côté du dos de la main (9), avec la partie médiane (5) sur le côté intérieur (10) du pouce (2) adjacent à la paume de la main (11), **caractérisée en ce que** l'orthèse (1) de pouce peut se déplier dans la région de la partie médiane (5) par un moyen d'articulation (27) disposé sur un étrier annulaire (3, 4) et peut être bloqué en position fermée par un moyen fermeture (28).

2. Orthèse de pouce (1) selon la revendication 1, **caractérisée en ce que** l'étrier annulaire inférieur (4) présente une surface d'appui (16) s'étendant jusqu'à l'articulation trapézo-métacarpienne (15).

3. Orthèse de pouce (1) selon l'une quelconque des revendications 1 ou 2, **caractérisée en ce que** la partie médiane (5) est conçue comme un rail de soutien (19) intégré élargi au moins en partie entre l'étrier annulaire (3) supérieur et l'étrier annulaire (4) inférieur.

4. Orthèse de pouce (1) selon la revendication 1, **caractérisée en ce que** la partie médiane (5) conçue d'un seul tenant est reliée au moins à un étrier annulaire (3, 4), de préférence à l'étrier annulaire (3) supérieur.

5. Orthèse de pouce (1) selon l'une quelconque des revendications 1 à 4, **caractérisée en ce que** les étriers annulaires (3, 4) sont conçus pour être ouverts afin d'assurer la mobilité unilatérale du pouce, du côté intérieur de celui-ci.

6. Orthèse de pouce (1) selon l'une quelconque des revendications 1 à 5, **caractérisée en ce que** l'orthèse (1) de pouce ou au moins des éléments de l'orthèse (1) de pouce est constitué de matière plastique, métal, matériau céramique ou matériau composite.

7. Orthèse (1) de pouce, selon l'une quelconque des revendications 1 à 6, **caractérisée en ce qu'**au moins des zones planes des éléments de l'orthèse (1) de pouce présentent des évidements filigranés (22) ou des déformations ergonomiques.
